(19) Europäisches Patentamt
European Patent Office
Office européen des brevets

(11) **EP 2 508 878 A1**

(12) **EUROPÄISCHE PATENTANMELDUNG**

(43) Veröffentlichungstag:
**10.10.2012  Patentblatt 2012/41**

(21) Anmeldenummer: **12162519.8**

(22) Anmeldetag: **30.03.2012**

(51) Int Cl.:
**G01N 27/416** (2006.01)     **G01N 33/18** (2006.01)
**C02F 1/46** (2006.01)       **C02F 1/467** (2006.01)
**C02F 1/72** (2006.01)        **C02F 1/78** (2006.01)

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(30) Priorität: **06.04.2011  AT 4872011**

(71) Anmelder: **pro aqua Diamantelektroden Produktion GmbH & Co KG**
**8712 Niklasdorf (AT)**

(72) Erfinder:
• **Schelch, Michael**
  **8600 Oberaich (AT)**
• **Staber, Wolfgang**
  **8600 Bruck an der Mur (AT)**
• **Hermann, Robert**
  **8600 Oberaich (AT)**
• **Wesner, Wolfgang**
  **1220 Wien (AT)**

(74) Vertreter: **Vinazzer, Edith**
  **Schönburgstraße 11/7**
  **1040 Wien (AT)**

(54) **Verfahren und Vorrichtung zur Bestimmung der Konzentration von Oxidationsmittel(n) in einer wässrigen Lösung**

(57)    Die Erfindung betrifft ein Verfahren und eine Vorrichtung zur Bestimmung der Konzentration von Oxidationsmittel(n) in einer in einem Hauptstrom strömenden wässrigen Lösung. Gemäß dem Verfahren wird in einen Bypass ein Teilstrom der wässrigen Lösung geleitet, wobei im Bypass die Differenz der Potentiale der wässrigen Lösung vor und nach zumindest teilweiser und/oder selektiver Zerstörung etwaig vorhandener Oxidationsmittel gemessen wird.

Die Vorrichtung weist einen Bypass zur Abzweigung und Rückführung eines Teilstromes der wässrigen Lösung, zumindest eine im Bypass vom Teilstrom der wässrigen Lösung durchströmte Eliminationseinheit (1) zur zumindest teilweisen und/oder selektiven Zerstörung des/der Oxidationsmittel und zwei Messelektroden (2, 2') zur Ermittlung der Differenz zwischen den Potentialen der wässrigen Lösung im Teilstrom vor und nach dem Durchströmen der Eliminationseinheit (1) auf.

Die Erfindung stellt daher ein zuverlässiges und einfach durchzuführendes Verfahren sowie ein entsprechende Vorrichtung zur Ermittlung der Konzentration von Oxidationsmittel in wässrigen Lösungen zur Verfügung.

Fig. 1

**EP 2 508 878 A1**

**Beschreibung**

Die Erfindung betrifft ein Verfahren und eine Vorrichtung zur Bestimmung der Konzentration von Oxidationsmittel(n) in einer in einem Hauptstrom strömenden wässrigen Lösung.

Es ist üblich, Oxidationsmittel, wie freies Chlor, Hypochlorit, Ozon, Wasserstoffperoxid und dergleichen zur Wasserdesinfektion, Wasserkonservierung oder Wasseraufbereitung einzusetzen. Sowohl Trinkwasser als auch Badewasser in Schwimmbecken, Schwimmteichen, Whirlpools, Wannenbädern und dergleichen, zur öffentlichen oder privaten Nutzung, werden mit Oxidationsmitteln aufbereitet. Auch in zahlreichen industriellen Anwendungen werden Oxidationsmittel bei der Aufbereitung von Prozesswässern verwendet. So werden Waschwässer in der Lebensmittelindustrie sowie Brauchwässer aus Regenwassersammelanlagen oder Abläufe von Kläranlagen mit Oxidationsmitteln versetzt, um ein Eliminieren von Keimen und ein Herabsetzen des chemischen Sauerstoffbedarfs (CSB) zu gewährleisten. Zusätzlich zu den erwähnten klassischen Oxidationsmitteln kommen in letzter Zeit auch Peroxide, Percarbonate und Persulfate vermehrt zum Einsatz. Chlordioxid wird auf Grund seiner guten Penetration in biologisches Material, insbesondere zur Bekämpfung von Biofilmen in Tanks und Rohrleitungen, eingesetzt. Darüber hinaus setzen sich zunehmend Verfahren durch, bei denen Oxidationsmittel bzw. Gemische von Oxidationsmitteln in situ durch anodische Oxidation von definierten Lösungen oder durch eine anodische Oxidation des zu behandelnden Medium selbst direkt hergestellt werden. Dieses Medium dient zudem als Anolyt. Kamen früher in erster Linie Mischoxidelektroden auf Titanbasis zum Einsatz, welche bevorzugt Chlor produzierten, so werden heute insbesondere Bor-dotierte Diamantelektroden eingesetzt, welche in Abhängigkeit vom Elektrolyt verschiedene Gemische an Oxidationsmitteln liefern, welche in ihrer Kombination eine vollständigere oxidative Umsetzungen und bessere Desinfektionsleistungen bereits bei geringen Konzentrationen der einzelnen Wirkstoffe zeigen.

Unabhängig davon, ob die Oxidationsmittel zudosiert oder vor Ort erzeugt werden, ist es wichtig, eine je nach Anwendung definierte Konzentration an Oxidationsmitteln einzustellen. Da Oxidationsmittel in Abhängigkeit vom chemischen Sauerstoffbedarf der jeweiligen wässrigen Lösung verbraucht werden und der jeweilige Verbrauch vom meist schwer vorhersehbaren Grad der Verschmutzung abhängig ist, ist eine zuverlässige automatisierbare Mess- und Regeleinheit zur Einstellung des Oxidationsmittelzusatzes von großem Interesse. Aus dem Stand der Technik sind unterschiedliche Verfahren zur Bestimmung der aktuellen Konzentration von Oxidationsmitteln in wässrigen Lösungen bekannt. Zu diesen Verfahren zählen photometrische Verfahren, insbesondere DPD Tests 1-3 zur Messung von freiem und gebundenem Chlor. Photometrische Verfahren können nur mit relativ hohem aparativen Aufwand automatisiert werden, sind mit einem ständigen Chemikalienverbrauch verbunden und erfassen nur bestimmte Oxidationsmittel. Des Weiteren gibt das Redoxpotential qualitative Informationen über das Vorhandensein bestimmter Oxidationsmittel. So wird ein Redoxpotential größer 700 mV als Indiz für eine ausreichende Chlorung von Schwimmbädern genutzt. Da unterschiedliche Oxidationsmittel unterschiedliche Redoxpotentiale bewirken, können quantitative Aussagen über die jeweils vorhandene Oxidationsmittelkonzentration aus solchen Messungen nicht abgeleitet werden. Chlor, Chlordioxid sowie Ozon sind ferner durch direkte Messung per Absorption im Infrarotbereich quantitativ messbar. Etwaige Trübungen stören jedoch die Messung. Andere Oxidationsmittel sind mit dieser Art von Messung nicht erfassbar.

Darüber hinaus sind amperometrische Verfahren bekannt, welche in Abhängigkeit vom eingesetzten Elektrodenmaterial, von eventuellen selektiven Membranen und der angelegten Spannung die Quantifizierung eines Oxidationsmittels oder mehrerer Oxidationsmittel gestatten. Es sind eine regelmäßige Kalibrierung der Messelektroden und ein regelmäßiger Austausch der Membranen erforderlich. Sind keine Membranen vorhanden, erfolgt durch den Stromfluss eine Abscheidung von Kalk oder Metallen, beispielsweise Eisen und Mangan, an den Messelektroden, sodass deren regelmäßige Reinigung erforderlich ist.

Der Erfindung liegt die Aufgabe zugrunde, ein einfaches, kostengünstig durchführbares und zuverlässiges Messverfahren und eine einfache sowie robuste Vorrichtung zur Bestimmung der Konzentration von Oxidationsmitteln oder Gemischen aus Oxidationsmitteln in wässrigen Lösungen zur Verfügung zu stellen.

Gelöst wird die gestellte Aufgabe erfindungsgemäß dadurch, dass vom Hauptstrom in einen Bypass ein Teilstrom der wässrigen Lösung geleitet wird, wobei im Bypass die Differenz der Potentiale der wässrigen Lösung vor und nach zumindest teilweiser und/oder selektiver Zerstörung etwaig vorhandener Oxidationsmittel gemessen wird.

Die Erfindung stellt somit ein zuverlässiges und einfach durchzuführendes Verfahren zur Ermittlung der Konzentration von Oxidationsmittel in wässrigen Lösungen zur Verfügung.

Zur Ermittlung der Potentialdifferenz sind lediglich zwei Messelektroden erforderlich. Es eignen sich vor allem Messelektroden erster Art, insbesondere Edelmetallelektroden, Titan-Mischelektroden, Graphitelektroden, Kohleelektroden oder Bor-dotierte Diamantelektroden. Die beiden Messelektroden können identisch oder unterschiedlich sein.

Auf besonders einfache und effektive Weise lässt/lassen sich das/die Oxidationsmittel beim Durchströmen einer Aktivkohleschüttung zumindest teilweise und/oder selektiv zerstören. Zusätzlich oder alternativ kann die wässrige Lösung zumindest einen Katalysator durchströmen, vorzugsweise aus Platin, Palladium oder Nickel, wobei der Katalysator auch ein selektiver Katalysator auf der Basis von Platin oder Palladium sein kann, welcher mit einem Katalysatorgift, wie Metalle oder Metallionen, vergiftet ist.

Je nach vorhandenem Oxidationsmittel kann es ferner vorteilhaft sein, die Aktivkohleschüttung oder den Katalysator auf ein kathodisches Potential zu legen, sodass durch Anlegen eines bestimmten Reduktionspotentials selektiv ein Oxidationsmittel, welches sich mit dem jeweiligen Potential reduzieren lässt, zerstört wird.

Ebenfalls alternativ oder zusätzlich kann im Rahmen des erfindungsgemäßen Verfahrens eine zumindest teilweise oder selektive Zerstörung von Oxidationsmittel durch ein Reduktionsmittel, beispielsweise $H_2$ erfolgen, welches an einer weiteren Elektrode erzeugt wird. Geeignete Reduktionsmittel lassen sich im Rahmen der Erfindung ebenfalls an weiteren Elektroden durch elektrochemische Auflösung erzeugen.

Im Rahmen der Erfindung ist es auch möglich, das/die Oxidationsmittel durch Zudosieren eines Reduktionsmittels, beispielsweise $H_2$, zumindest teilweise und/oder selektiv zu eliminieren.

Als weitere zusätzliche oder alternative Maßnahmen zur Zerstörung des/der Oxidationsmittel kommen UV-Strahlung, Hitzeeinwirkung oder Ultraschall in Frage.

Die Erfindung betrifft ferner eine Vorrichtung zur Bestimmung der Konzentration von Oxidationsmitteln in einer in einem Hauptstrom strömenden wässrigen Lösung. Diese Vorrichtung umfasst zumindest eine im Bypass vom Teilstrom der wässrigen Lösung durchströmte Eliminationseinheit zur zumindest teilweisen und/oder selektiven Zerstörung des/der Oxidationsmittel und zwei Messelektroden zur Ermittlung der Differenz zwischen den Potentialen der wässrigen Lösung im Teilstrom vor und nach dem Durchströmen der Eliminationseinheit. Bei der erfindungsgemäßen Vorrichtung handelt es sich daher um eine einfach und zweckmäßig aufgebaute und zuverlässig arbeitende Vorrichtung.

Die Vorrichtung wird mit einer entsprechenden Messelektronik versehen oder mit einer solchen gekoppelt.

Im einfachsten Fall ist die Eliminationseinheit ein Rohr, ein Schlauch oder dergleichen, welcher eine Aktivkohleschüttung und/oder zumindest einen Katalysator enthält. Dieser Katalysator kann insbesondere ein Platin-, Palladium- oder Nickelkatalysator oder ein selektiver Katalysator auf Basis von Platin oder Palladium sein, welcher mittels eines Katalysatorgiftes vergiftet ist.

Zusätzlich oder alternativ weist die Vorrichtung zumindest eine Dosiereinrichtung zur Zudosierung von Reduktionsmittel in die Eliminationseinheit auf.

Die Vorrichtung kann eine weitere Elektrode zum Anlegen eines kathodischen Potentials an die Aktivkohleschüttung aufweisen. Die Vorrichtung kann ferner erfindungsgemäß eine vor der Eliminationseinheit positionierte Elektrode zur Erzeugung von Wasserstoff aufweisen. Bei einer Ausführungsform der Erfindung weist die Vorrichtung mindestens eine weitere, ebenfalls vor der Eliminationseinheit positionierte Elektrode auf, welche durch elektrochemische Auflösung ein Reduktionsmittel freisetzt. Die zu den weiteren Elektroden gegengeschalteten Anoden sind in Strömungsrichtung nach der zweiten Messelektrode positioniert.

Zum Einbringen des Teilstromes in die Vorrichtung und Aufrechterhaltung eines konstanten Durchflusses können unterschiedliche Maßnahmen getroffen werden. Einfach und zuverlässig ist das Vorsehen einer elektrischen Pumpe in der Vorrichtung. Es eigenen sich jedoch auch hydraulische Komponenten, wie Ventile, Drosseln, Drosselklappen, Blenden und dergleichen.

Eine sehr einfache und zuverlässige Möglichkeit, einen konstanten Durchfluss sicherzustellen, ist der Einsatz eines Flow-Controllers, welcher vor der Eliminationseinheit eingebaut ist.

Messsignale der Messelektronik werden gemäß der Erfindung zur Regelung einer Oxidationsmitteldosiereinrichtung oder einer Einrichtung zur Insitu-Erzeugung von Oxidationsmittel verwendet. Die Vorrichtung wird ferner zweckmäßigerweise als austauschbare Einheit konzipiert, welche zumindest die Messelektroden und die Eliminationseinheit umfasst. Die Messelektronik ist vorteilhafterweise zumindest teilweise in diese austauschbare Einheit integriert.

Weitere Merkmale, Vorteile und Einzelheiten der Erfindung werden nun anhand der schematischen Zeichnung, die Ausführungsbeispiele darstellt, näher beschrieben. Dabei zeigen

Fig. 1 eine Ansicht einer Ausführungsvariante einer erfindungsgemäßen Vorrichtung,

Fig. 2 eine zweite Ausführungsvariante einer erfindungsgemäßen Vorrichtung,

Fig. 3 eine Alternative der in Fig. 2 gezeigten Variante und

Fig. 4 und Fig. 5 grundsätzliche Möglichkeiten der Anordnung bzw. Einbindung einer erfindungsgemäßen Vorrichtung in einen Wasserkreislauf.

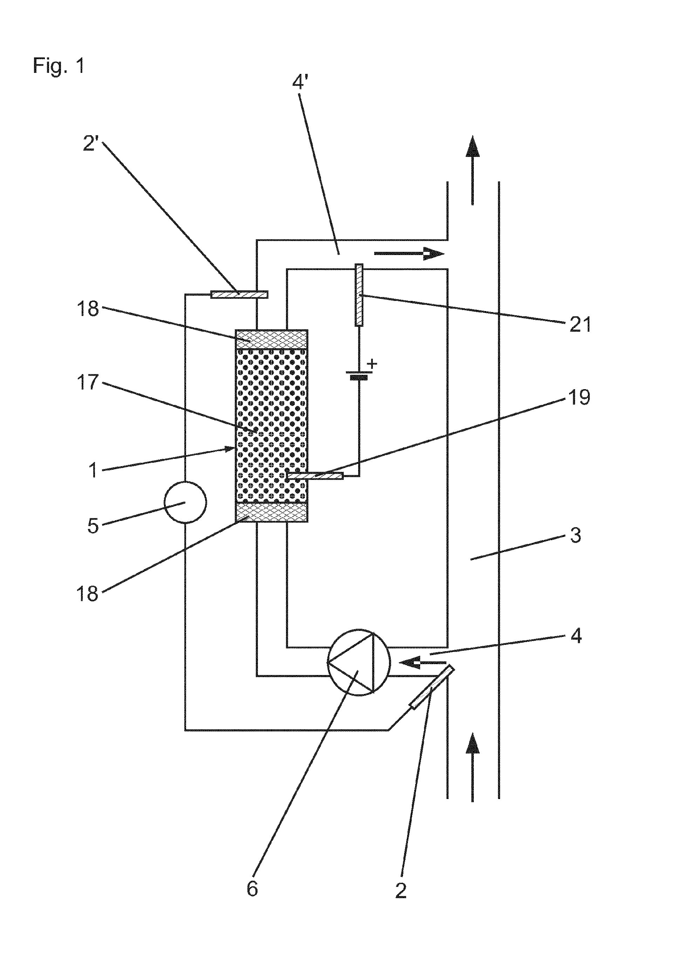

Die erfindungsgemäße Vorrichtung wird als Bypass in einem Wasserkreislauf oder in einem Wasserleitungssystem, in welchem die Konzentration des/der im Wasser enthaltenen Oxidationsmittel(s) oder Oxidationsmittelgemische festgestellt werden soll, eingebaut und an ein Durchflussrohr 3 oder dergleichen, welches von dem zu messenden Wasser durchströmt wird, angeschlossen. Wie Fig. 1 bis Fig. 3 zeigen, umfasst die erfindungsgemäße Vorrichtung ein vom Durchflussrohr 3 abzweigendes Rohr 4 und ein in einem Abstand von diesem in das Rohr 3 einmündendes Rohr 4'. Die Rohre 4, 4' können insbesondere konstante und übereinstimmende Querschnitte aufweisen, welche deutlich geringer sind als der Querschnitt des Rohres 3. Von Bedeutung ist vorrangig der Wasserdurchfluss im Bypassstrom. Die

**EP 2 508 878 A1**

erfindungsgemäße Vorrichtung wird je nach Anwendung auf einen Durchfluss zwischen 0,1 1/Stunde bis 201/Stunde ausgelegt, bei einem Einsatz im Rahmen der Wasseraufbereitung von Poolwasser auf einen Durchfluss zwischen 0,5 1/Stunde und 21/Stunde. Zwischen dem abzweigenden und dem einmündenden Rohr 4, 4' ist eine Eliminationseinheit 1 eingebaut, durch welche der Teilstrom, welcher von dem im Rohr 3 fließenden Hauptstrom abgezweigt wird, durchgeleitet wird. Die Strömungsrichtungen des Hauptstromes und des Teilstromes sind in Fig. 1 bis Fig. 3 durch Pfeile angedeutet. Die erfindungsgemäße Vorrichtung weist, in Strömungsrichtung betrachtet, sowohl vor der Eliminationseinheit 1 als auch nach der Eliminationseinheit 1 eine Messelektrode 2, 2' auf, welche ins Wasser eintaucht und mit einer Messelektronik 5 verbunden sind. Die Messelektrode 2 kann dabei an beliebiger Stelle in den Teilstrom im abzweigenden Rohr 4 oder in den Hauptstrom im Rohr 3 eintauchen. Die Messelektrode 2' befindet sich nach der Eliminationseinheit 1 an beliebiger Stelle im einmündenden Rohr 4'. Die Anordnung der erfindungsgemäßen Vorrichtung als Bypass sorgt für den für den Messvorgang erforderlichen geschlossenen Stromkreis.

Bei der in Fig. 1 gezeigten Ausführungsvariante sorgt eine im abzweigenden Rohr 4 positionierte Pumpe 6, deren Förderkapazität auf den gewünschten Durchfluss abgestimmt ist, für eine konstante Durchströmung der Eliminationseinheit 1, um in Abhängigkeit von der Kapazität der Eliminationseinheit 1, wie noch beschrieben wird, eine vollständige oder eine definierte (prozentuell konstante) Eliminierung des/der Oxidationsmittel(s) nach dem Passieren der Eliminationseinheit 1 der Oxidationsmittel sicherzustellen.

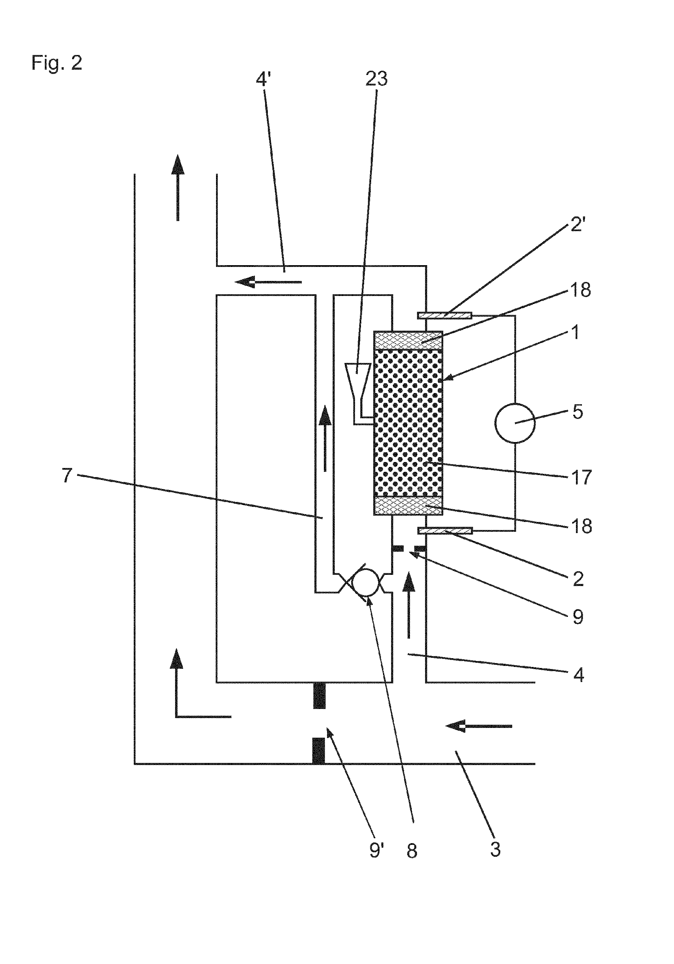

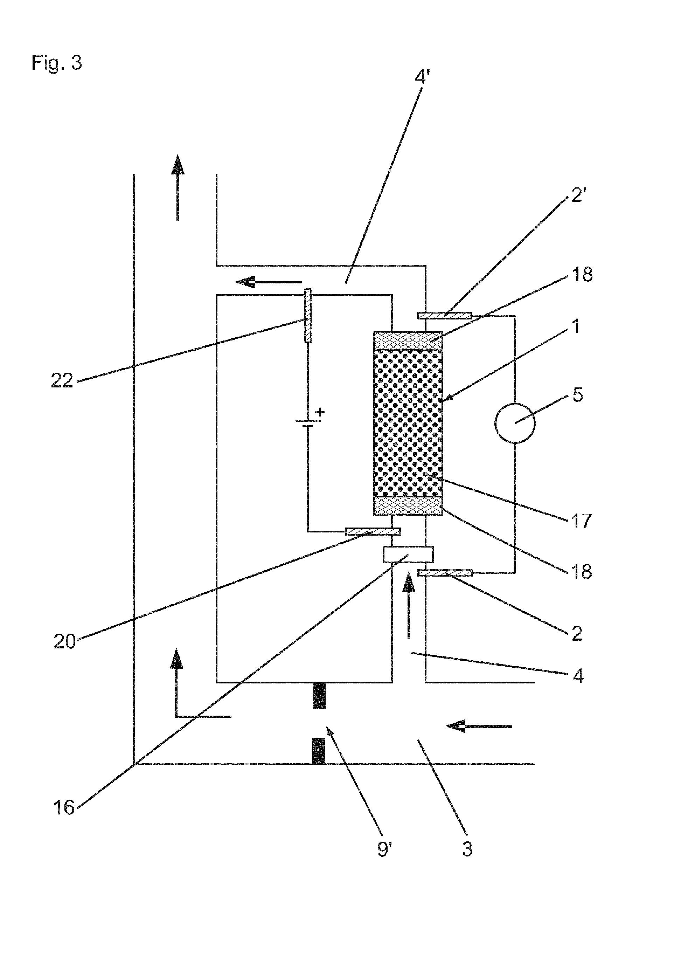

Ein konstanter Durchfluss bzw. konstanter Teilstrom kann alternativ oder ergänzend durch andere mechanische (hydraulische) oder elektrische Einrichtungen gewährleistet werden. In Fig. 2 erfolgt mittels hydraulischer Einrichtungen/Komponenten ein konstanter Durchfluss bzw. eine konstante Durchströmung der Eliminationseinheit 1. Parallel zur Eliminationseinheit 1 ist eine Bypassleitung 7 vorgesehen, welche vor der Eliminationseinheit 1 abzweigt und nach dieser in das Rohr 4' einmündet. Die Bypassleitung 7 weist einen insbesondere konstanten Querschnitt auf, welcher geringer ist, als jener der Rohre 4, 4'. Unmittelbar nach der Abzweigungsstelle vom Rohr 4 befindet sich in der Bypassleitung 7 ein Ventil 8, welches sich bei Überschreiten eines gewissen Druckes im Teilstrom, der durch das abzweigende Rohr 4 strömt, öffnet. Als weitere, eine konstante Durchströmung der Eliminationseinheit 1 sicherstellende Maßnahme kann im Rohr 4 nach der Abzweigung der Bypassleitung 7 eine den Querschnitt des Rohres 4 verkleinernde Einrichtung, beispielsweise eine Drossel 9 oder Blende eingebaut werden. Des Weiteren ist im vom Hauptstrom durchflossenen Rohr 3 unmittelbar nach dem abzweigenden Rohr 4 eine Drossel 9' oder Blende eingebaut, die für eine Druckerhöhung und damit für ein Zustandekommen des Teilstromes im abzweigenden Rohr 4 sorgt. Es eignen sich an sich bekannte Drosseln bzw. einstellbare Drosselklappen. Zusätzlich oder alternativ zu Drosselklappen oder Ventilen können Lochscheiben, Lochplatten und dergleichen, welche die Querschnitte im Rohr 3 und/oder im Rohr 4 entsprechend verkleinern, eingesetzt werden. Bei der in Fig. 3 gezeigten, alternativen Ausführungsform sorgt ebenfalls eine Drossel 9' im Rohr 3 für eine Druckerhöhung und ein Abzweigen eines Teilstromes ins Rohr 4. Ein vor der Eliminationseinheit 1 eingebauter Flow-Controller 16 an sich bekannter Bauart sorgt für eine konstante Durchflussmenge.

Die beiden Messelektroden 2, 2' ermitteln die Potentialdifferenz zwischen dem Wasser vor dem Eintritt in die Eliminationseinheit 1 und dem Wasser nach der Passage der Eliminationseinheit 1. Die Auswertung der Messwerte erfolgt in der Messelektronik 5. Die Eliminationseinheit 1 eliminiert das/die im Wasser enthaltene(n) Oxidationsmittel zur Gänze oder teilweise und/oder selektiv. Die ermittelte Potentialdifferenz ist den Oxidationsäquivalenten proportional. Wird beispielsweise durch das Einstellen einer höheren Durchflussmenge an Wasser durch die Eliminationseinheit 1 nur ein bestimmter Prozentsatz an Oxidationsmittel eliminiert bzw. abgebaut, bleibt die Proportionalität des Signals zur Gesamtmenge an Oxidantien erhalten.

In jedem Fall ist eine einmalige Eichung der Vorrichtung vorzunehmen. Soll eine nicht vollständige Zerstörung des/der Oxidationsmittel erfolgen ist ferner vorab eine Messung mit einer bekannten Methode durchzuführen, um das Ausmaß der prozentuellen Zerstörung festzustellen. Wie weiter unten noch beschreiben, ist dies für die Regelung einer etwaigen Dosiereinrichtung für Oxidationsmittel von Bedeutung.

Alternativ dazu kann eine interne Eichung der Vorrichtung durch die Extrapolation des prozentuellen Abbaus bei unterschiedlichen Durchflussgeschwindigkeiten erfolgen. Dazu wird die Anordnung mit einer oxidationsmittelhaltigen wässrigen Lösung mit verschiedenen Durchflussgeschwindigkeiten beschickt. Daraus wird eine Eichkurve "Durchfluss gegen Potential" ermittelt. Aus dieser Kurve wird die theoretische Durchflussgeschwindigkeit bei 100% Elimination bestimmt. Diesem Wert entspricht ein Potential, sodass es möglich ist, direkt über die Nernstsche Gleichung die Aktivität der zu bestimmenden Oxidationsmittel zu berechnen:

$$\Delta E = \frac{RT}{z_e F} \ln \frac{a_g}{a_k}$$

$a_g$ steht für die Aktivität der größeren Konzentration vor der Eliminationseinheit 1,
$a_k$ steht für die Aktivität der kleineren Konzentration nach der Eliminationseinheit 1.

In guter Näherung kann bei bekannten Systemen die Aktivität der Oxidationsmittel der Konzentration gleichgesetzt oder zumindest proportional gesetzt werden.

Im Bereich von 22°C bis 26°C kann RT/F gleich 0,059 gesetzt werden. Z wird mit 1 angenommen, wenn es darum geht die Oxidationsäquivalente zu bestimmen.

Die Eliminierung bzw. Zerstörung des bzw. der Oxidationsmittel(s) im Wasser beim Durchfließen der Eliminationseinheit 1 kann mit verschiedenen Maßnahmen erreicht werden. Im einfachsten Fall besteht die Eliminationseinheit 1 aus einem Rohr, Schlauch oder dergleichen, welches bzw. welcher mit einer Schüttung 17 aus Aktivkohle gefüllt ist. Es eignet sich beispielsweise Aktivkohle in Form von Partikeln einer Größe zwischen 0,5 mm und 6 mm. Das Rohr, der Schlauch oder dergleichen wird beidseitig mit einem wasserdurchlässigen Stoppel 18 verschlossen und weist beispielsweise eine Länge von einigen Zentimetern bis zu einigen Metern (bei Schlauchausführung) und einen Durchmesser von 10 mm bis 5 cm auf. Aktivkohle ist in Folge ihrer hochporösen Struktur und ihrer katalytischen Eigenschaften in der Lage, Oxidationsmittel zu zerstören. Die katalytische Wirkung kann dadurch unterstützt werden, dass weitere Katalysatoren, wie Palladium, Platin, Rhodium, Ruthenium, Kobalt, Eisen, Kupferchromit und Zinkchromit, in die Eliminationseinheit 1 eingebracht werden, beispielsweise als Beschichtung bzw. Ummantelung von Aktivkohlepartikeln. Darüber hinaus ist es möglich, die Reduktionsleistung zu verbessern, indem die Aktivkohleschüttung auf ein kathodisches Potential gelegt wird, indem beispielsweise eine Elektrode 19 (Fig. 1), etwa ein Kohlestab, in die Schüttung 17 eingebracht wird. Alternativ oder zusätzlich kann vor der Eliminationseinheit 1 Wasserstoff an einer im Wasser positionierten Elektrode 20 (Fig. 3) (Kathode) erzeugt werden, wobei der Wasserstoff mit dem Wasser in die Eliminationseinheit 1 transportiert wird und dort an der Oberfläche der Aktivkohle mit dem/den Oxidationsmittel(n) reagiert. Die zu den Elektroden 19, 20 gegengeschalteten Anoden 21, 22 befinden sich beispielsweise in Strömungsrichtung nach der Messelektrode 2' im Rohr 4', sodass die an diesen gebildeten Oxidationsmittel den Messwert nicht beeinflussen.

Bei anderen Ausführungsformen enthält die Eliminationseinheit 1 zumindest einen Katalysator, wie Palladium, Nickel, Platin, Rhodium, Ruthenium, Kobalt, Eisen, Kupferchromit und Zinkchromit. Die Katalysatoren werden beispielsweise als entsprechend beschichtetes Trägermaterial in die Einheit 1 eingebracht. Je nach Katalysatormaterial wird/werden in der Eliminationseinheit 1 selektiv ein/mehrere im Wasser vorhandene(n) Oxidationsmittel abgebaut. Dabei kann eine erfindungsgemäße Vorrichtung auch mehrer Eliminationseinheiten 1 mit jeweils unterschiedlichen Katalysatoren enthalten, die in Serie aneinander gereiht sind, welche vom Teilstrom nacheinander durchlaufen werden. Alternative oder zusätzliche Maßnahmen zu Katalysatoren oder Aktivkohle sind diverse Behandlungen des Wassers beim Durchlaufen der Eliminationseinheit 1, insbesondere eine definierte Einwirkung von Hitze, eine Behandlung mit UV-Strahlung oder mit Ultraschall.

Das/die Oxidationsmittel im Wasser kann/können ferner durch ein unmittelbares Einbringen von Wasserstoff zerstört werden. Der Wasserstoff kann beispielsweise aus einem Behälter 23 (Fig.3) in die Eliminationseinheit 1, welche auch Aktivkohle und/oder einen/mehrere der erwähnten Katalysatoren enthalten kann, über eine Dosiereinrichtung eingebracht werden.

In der Eliminationseinheit 1 können ferner selektive Katalysatoren auf der Basis von Platin oder Palladium verwendet werden, welche mittels eines Katalysatorgiftes, wie Metalle oder Metallionen, beispielsweise Ca, Mg, Pb, vergiftet sind, sodass sich selektiv bestimmte Oxidationsmittel abbauen lassen.

Wie bereits erwähnt kann in der Eliminationseinheit 1 mindestens eine Kathode angebracht werden, welche durch Anlegen definierter Reduktionspotentiale selektiv Oxidationsmittel, welche sich mit dem jeweils angelegten Potential reduzieren lassen, zerstört.

Bei der Auswahl des Elektrodenmaterials für die Messelektroden 2, 2' zur Messung der Potentialdifferenz ist darauf zu achten, dass die Messflüssigkeit keine Reaktionen verursacht, welche das Eigenpotential der Messelektroden 2, 2' verändern. Bevorzugt werden beide Messelektroden 2, 2' identisch ausgeführt und es werden als Messelektroden 2, 2' Elektroden erster Art eingesetzt, dies sind Elektroden, deren Potential direkt von der Konzentration der Ionen in der Messflüssigkeit abhängt. Geeignete Elektrodenmaterialien sind insbesondere Edelmetalle, wie Platin, Iridium, Gold oder Silber. Des weiteren kommen dimensionsstabile Elektroden, wie Titan-Mischoxidelektroden oder Elektroden aus Titan-Iridiumoxid, in Frage, ferner Elektrodenmaterialien wie Kohle, Kohlefaser, Graphit, Glassy-Carbon sowie Bor-dotierte Diamantelektroden oder dotierte Siliziumelektroden.

An weiteren Elektroden, beispielsweise der oben erwähnten Elektrode 20, kann ein Reduktionsmittel, beispielsweise $H_2$, durch Elektrolyse der Messflüssigkeit hergestellt werden, welches direkt oder in Kombination mit den Katalysatoren in der Eliminationseinheit 1 in der Lage ist, ein Oxidationsmittel selektiv, teilweise oder vollständig zu reduzieren. Weitere, analog angeordnete, nicht dargestellte Elektroden können ferner durch elektrochemische Auflösung Reduktionsmittel, beispielsweise reaktive Metallionen, wie Eisen (I), Eisen (II), Zink (I), Kupfer (I), Aluminium (I), Aluminium (II), Magnesium (I) freisetzen, welche Metallionen direkt oder in Kombination mit dem/den Katalysator(en) in der Eliminationseinheit 1 in der Lage sind, Oxidationsmittel selektiv, teilweise oder vollständig zu reduzieren. Wie bereits erwähnt, kann alternativ ein Reduktionsmittel, beispielsweise $H_2$, in die Eliminationseinheit 1 zudosiert werden, welches ebenfalls das/die Oxidationsmittel selektiv, teilweise oder vollständig reduziert.

Um eine Biofilmbildung auf den Messelektroden 2, 2' zu verringern oder um etwaige Ablagerungen auf den Messelektroden 2, 2' zu entfernen, kann in regelmäßigen Zeitintervallen die kontinuierliche Messung für wenige Minuten unterbrochen werden und eine Spannung von mehreren Volt an die Messelektroden 2, 2' angelegt werden, sodass die gasförmigen Elektrolyseprodukte der Messflüssigkeit, welche die Elektroden 2, 2' umgibt, (Wasserstoff an der Kathode, Sauerstoff an der Anode) die Ablagerungen von den Elektrodenoberflächen absprengen, sodass deren reaktive Oberflächen zur Messung der Potentialdifferenz erhalten bleibt.

Gemessen wird ein Signal im Millivoltbereich über einen hochohmigen Messeingang einer Verstärkerschaltung, in bevorzugter Weise wird ein Feldeffekttransistor oder ein Operationsverstärker mit einem entsprechend hochohmigen Eingang mit den Elektroden 2, 2' verbunden. Diese Komponenten befinden sich in der Messelektronik 5. Die Spannung an den Elektroden 2 , 2' ist Null, wenn kein Oxidationsmittel in der Eliminationseinheit 1 zerstört wird. Ändert sich die Konzentration an Oxidationsmittel(n) vor und nach dem Durchlaufen der Eliminationseinheit 1, ändert sich das gemessene Potential proportional zur Konzentrationsänderung (höhere Konzentration vor der Eliminationseinheit 1 bewirkt eine größere Proportionaldifferenz), wobei die Proportionalität nicht notwendigerweise linear ist.

Erfindungsgemäße Vorrichtungen werden vorzugsweise als austauschbare Einheiten konzipiert und umfassen jeweils zumindest die beiden Messelektroden 2, 2', eine Eliminationseinheit 1 und die zugehörigen Zu- und Ableitungsrohre 4, 4'. Die Messelektronik 5 kann komplett oder zum Teil in die austauschbare Einheit integriert sein. Von der Messelektronik 5 wird ein Messsignal, ein kodiertes Messsignal (frequenzmoduliert, digital oder als mA-Schleife) oder ein Regelsignal an eine externe Steuereinheit übermittelt, um eine gezielte Zudosierung von Oxidationsmittel oder die Produktion von Oxidationsmittel zu regeln oder zu steuern.

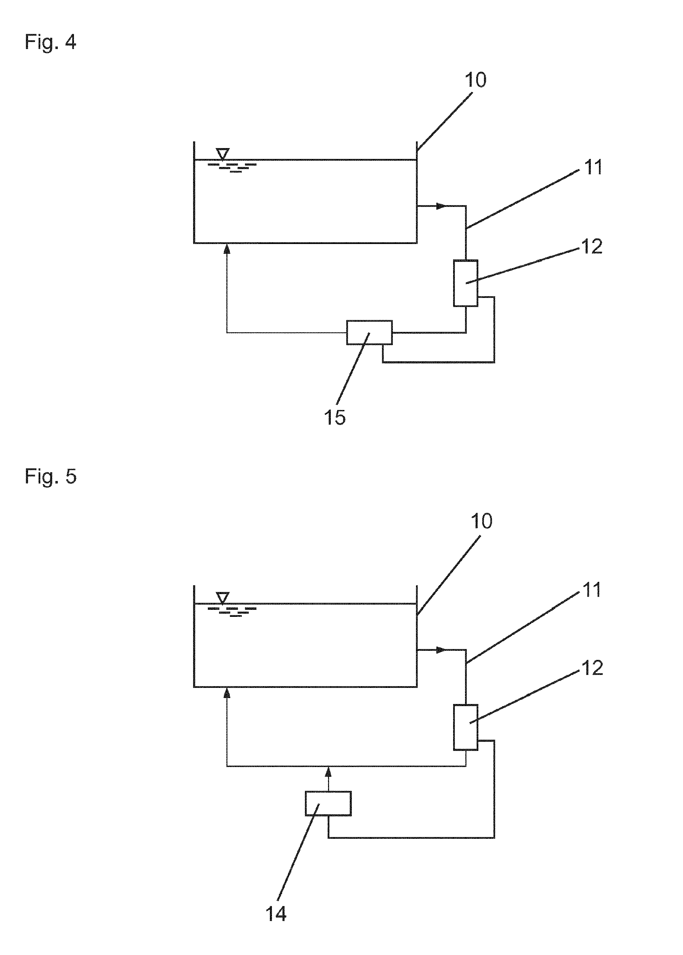

Fig. 4 und Fig. 5 zeigen grundsätzliche Anordnungen einer erfindungsgemäßen Vorrichtung 12, welche als austauschbare Einheit konzipiert ist, in einem geschlossenen Wasserkreislauf 11, welcher beispielsweise ein Wasserkreislauf zur Wasseraufbereitung von Schwimmbadwasser aus einem Becken 10 oder Wasser aus Whirlpools ist. Im Wasserkreislauf 11 ist eine erfindungsgemäße Vorrichtung 12 als austauschbare Einheit eingebaut. Alternativ kann die Vorrichtung unmittelbar an das Becken 10 angeschlossen werden. Die von der in die Vorrichtung 12 integrierten Messelektronik erfassten Werte werden, wie bereits erwähnt, zur Regelung einer Dosiereinheit 14 (Fig. 5) zur Dosierung von Oxidationsmittel verwendet. Wie es Fig. 4 zeigt, können die Messwerte auch zur Regelung einer Einrichtung 15 zur Oxidationsmittelproduktion mittels anodischer Oxidation verwendet werden.

Die erfindungsgemäße Vorrichtung sowie die über diese erfolgte Regelung einer Oxidationsmitteldosierung und/oder einer Oxidationsmittelproduktion kann ferner bei sogenannten Hot-Tubs, bei Wannenbädern, in der Prozesswasseraufbereitung oder der Trinkwasseraufbereitung eingesetzt werden.

**Bezugsziffernliste**

| | | |
|---|---|---|
| 1 | ...................... | Eliminationseinheit |
| 2, 2' | .................. | Messelektroden |
| 3 | ...................... | Durchflussrohr |
| 4 | ...................... | abzweigendes Rohr |
| 4' | ...................... | einmündendes Rohr |
| 5 | ...................... | Messelektronik |
| 6 | ...................... | Pumpe |
| 7 | ...................... | Bypassleitung |
| 8 | ...................... | Ventil |
| 9, 9' | .................. | Drosselklappe |
| 10 | ..................... | Becken |
| 11 | ..................... | Wasserkreislauf |
| 12 | .................... | Vorrichtung |
| 14 | ..................... | Dosiereinheit |
| 15 | ..................... | Einrichtung |
| 16 | .................... | Flow-Controller |
| 17 | .................... | Schüttung |
| 18 | ..................... | Stoppel |
| 19, 20 | ............... | Elektrode (Kathode) |
| 21, 22 | ............... | Elektrode (Anode) |
| 23 | ..................... | Behälter |

**Patentansprüche**

1. Verfahren zur Bestimmung der Konzentration von Oxidationsmittel(n) in einer in einem Hauptstrom strömenden wässrigen Lösung,
**dadurch gekennzeichnet,**

**dass** vom Hauptstrom in einen Bypass ein Teilstrom der wässrigen Lösung geleitet wird, wobei im Bypass die Differenz der Potentiale der wässrigen Lösung vor und nach zumindest teilweiser und/oder selektiver Zerstörung etwaig vorhandener Oxidationsmittel gemessen wird.

2. Verfahren nach Anspruch 1, **dadurch gekennzeichnet, dass** die Potentialdifferenz mittels zweier Messelektroden (2, 2') ermittelt wird, welche vorzugsweise Elektroden erster Art sind, insbesondere Edelmetallelektroden, Titan-Mischoxidelektroden, Graphitelektroden, Kohleelektroden oder Bor-dotierte Diamantelektroden.

3. Verfahren nach Anspruch 1, **dadurch gekennzeichnet, dass** die im Teilstrom fließende wässrige Lösung eine Aktivkohleschüttung durchströmt.

4. Verfahren nach Anspruch 1 oder 3, **dadurch gekennzeichnet, dass** die im Teilstrom fließende wässrige Lösung zumindest einen Katalysator, insbesondere aus Platin, Palladium oder Nickel, durchströmt, wobei vorzugsweise der Katalysator ein selektiver Katalysator auf der Basis von Platin oder Palladium ist, welcher mit einem Katalysatorgift, wie Metalle und Metallionen, vergiftet ist.

5. Verfahren nach Anspruch 3 oder 4, **dadurch gekennzeichnet, dass** die Aktivkohleschüttung oder der Katalysator auf ein kathodisches Potential gelegt wird, wobei durch Anlegen eines bestimmten Reduktionspotentials selektiv ein Oxidationsmittel, welches sich mit den jeweils angelegten Potential reduzieren lässt, zerstört wird.

6. Verfahren nach einem der Ansprüche 1 bis 5, **dadurch gekennzeichnet, dass** das/die Oxidationsmittel durch ein Reduktionsmittel, beispielsweise $H_2$, welches an einer zusätzlichen Elektrode (20) (Kathode) erzeugt wird oder kontinuierlich zudosiert wird, zumindest teilweise und/oder selektiv zerstört wird bzw. werden.

7. Verfahren nach einem der Ansprüche 1 bis 5, **dadurch gekennzeichnet, dass** die/das Oxidationsmittel durch ein Reduktionsmittel, welches an einer zusätzlichen Elektrode durch elektrochemische Auflösung erzeugt wird, oder unter Einwirkung von UV-Strahlung, Hitze oder Ultraschall zumindest teilweise und/oder selektiv zerstört wird bzw. werden.

8. Vorrichtung zur Bestimmung der Konzentration von Oxidationsmittel(n) in einer in einem Hauptstrom strömenden wässrigen Lösung,
**dadurch gekennzeichnet,**
**dass** sie einen Bypass zur Abzweigung und Rückführung eines Teilstromes der wässrigen Lösung,

zumindest eine im Bypass vom Teilstrom der wässrigen Lösung durchströmte Eliminationseinheit (1) zur zumindest teilweisen und/oder selektiven Zerstörung des/der Oxidationsmittel
und zwei Messelektroden (2, 2') zur Ermittlung der Differenz zwischen den Potentialen der wässrigen Lösung im Teilstrom vor und nach dem Durchströmen der Eliminationseinheit (1) aufweist.

9. Vorrichtung nach Anspruch 12, **dadurch gekennzeichnet, dass** sie eine Messelektronik (5) aufweist.

10. Vorrichtung nach Anspruch 8 oder 9, **dadurch gekennzeichnet, dass** die Eliminationseinheit (1) ein Rohr, Schlauch oder dergleichen ist, welcher bzw. welches eine Aktivkohleschüttung (17) und/oder mindestens einen Katalysator enthält, welcher vorzugsweise ein Platin-, Palladium- oder Nickel-Katalysator oder ein selektiver Katalysator auf Basis von Platin oder Palladium ist, welcher mittels eines Katalysatorgiftes vergiftet ist.

11. Vorrichtung nach einem der Ansprüche 8 bis 10, **dadurch gekennzeichnet, dass** sie eine weitere Elektrode (19) zum Anlegen eines kathodischen Potentials an die Aktivkohleschüttung (17) aufweist.

12. Vorrichtung nach einem der Ansprüche 8 bis 11, **dadurch gekennzeichnet, dass** sie mindestens eine weitere, vor der Eliminationseinheit (1) positionierte Elektrode (20) (Kathode) aufweist, welche Wasserstoff erzeugt oder welche durch elektrochemische Auflösung ein Reduktionsmittel freisetzt aufweist, wobei vorzugsweise die gegengeschaltete Anode (21, 22) in Strömungsrichtung nach der zweiten Messelektrode (2') positioniert ist.

13. Vorrichtung nach einem der Ansprüche 8 bis 12, **dadurch gekennzeichnet, dass** sie eine Pumpe (6) zum Einbringen des Teilstromes und/oder hydraulische Komponenten, wie Ventile (8), Drosseln (9), Blenden und dergleichen, zum Einbringen und zum Aufrechterhalten eines konstanten Teilstromes und/oder einen der Eliminationseinheit (1) vorgesetzten Flow-Controller enthält.

14. Vorrichtung nach einem der Ansprüche 8 bis 13, **dadurch gekennzeichnet, dass** die Messelektronik (5) Messsignale zur Verfügung stellt, welche zur Regelung einer Oxidationsmittel-Dosiereinrichtung (14) oder einer Einrichtung (15) zur in situ Erzeugung von Oxidationsmittel verwendet werden.

15. Vorrichtung nach einem der Ansprüche 8 bis 14, **dadurch gekennzeichnet, dass** zumindest die Messelektroden (2, 2') und die Eliminationseinheit (1) in einer austauschbaren Einheit zusammengefasst sind, in welche vorzugsweise auch zumindest Teile der Messelektronik integriert sind.

Fig. 1

Fig. 2

Fig. 3

Fig. 4

Fig. 5

Europäisches
Patentamt

European
Patent Office

Office européen
des brevets

**EUROPÄISCHER RECHERCHENBERICHT**

Nummer der Anmeldung

EP 12 16 2519

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 4 533 440 A (KIM BANG M [US]) 6. August 1985 (1985-08-06) * Abbildungen * ----- | 1,8,9 | INV. G01N27/416 G01N33/18 C02F1/46 C02F1/467 |
| X | DE 27 52 538 A1 (SICK KG OTTO) 31. Mai 1979 (1979-05-31) * Seite 10; Abbildungen * ----- | 1,2,8 | |
| X | US 2002/152036 A1 (MARTIN ROY [US]) 17. Oktober 2002 (2002-10-17) * das ganze Dokument * ----- | 1,7-9,14 | ADD. C02F1/72 C02F1/78 |
| X | US 6 391 256 B1 (MOON JEON SOO [KR] ET AL) 21. Mai 2002 (2002-05-21) * Abbildung 1; Beispiele 1,3 * ----- | 1,3-15 | |
| A | Donau Carbon: "Aktivkohle und ihre Anwendung", , 1. Januar 2010 (2010-01-01), XP55034966, Gefunden im Internet: URL:http://www.donau-carbon.com/Downloads/ aktivkohle.aspx [gefunden am 2012-08-08] * Seite 12, linke Spalte * ----- | 1-15 | |
| | | | RECHERCHIERTE SACHGEBIETE (IPC) G01N C02F |

Der vorliegende Recherchenbericht wurde für alle Patentansprüche erstellt

| Recherchenort | Abschlußdatum der Recherche | Prüfer |
|---|---|---|
| Den Haag | 10. August 2012 | Kraus, Leonie |

EPO FORM 1503 03.82 (P04C03)

## EP 2 508 878 A1

**ANHANG ZUM EUROPÄISCHEN RECHERCHENBERICHT
ÜBER DIE EUROPÄISCHE PATENTANMELDUNG NR.**

EP 12 16 2519

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

10-08-2012

| Im Recherchenbericht angeführtes Patentdokument | Datum der Veröffentlichung | Mitglied(er) der Patentfamilie | Datum der Veröffentlichung |
|---|---|---|---|
| US 4533440 A | 06-08-1985 | KEINE | |
| DE 2752538 A1 | 31-05-1979 | KEINE | |
| US 2002152036 A1 | 17-10-2002 | CA 2438292 A1<br>DE 60226134 T2<br>EP 1390731 A2<br>US 2002152036 A1<br>US 2002153325 A1<br>WO 02101372 A2 | 19-12-2002<br>28-05-2009<br>25-02-2004<br>17-10-2002<br>24-10-2002<br>19-12-2002 |
| US 6391256 B1 | 21-05-2002 | KEINE | |